# EUROPEAN PATENT APPLICATION

(11) **EP 1 650 204 A1**
(43) Date of publication of application: **26.04.2006**
(21) Application number: 04747845.8
(22) Date of filing: 23.07.2004
(51) Int. Cl.: C07D 401/14, C07D 401/04, A61K 31/444, A61K 31/4439, A61P 19/06

(54) **PROCESS FOR PRODUCING 1,2,4-TRIAZOLE COMPOUND AND INTERMEDIATE THEREFOR**

(30) Priority: 24.07.2003 JP 2003201286
(71) Applicant: Fujiyakuhin Co., Ltd., Saitama-shi, Saitama 330-0854 (JP)
(72) Inventor: NAKAMURA, Hiroshi, c/o Fujiyakuhin Co., LTD., Saitama-shi, Saitama 331-0047 (JP); UDA, Junichiro, c/o Fujiyakuhin Co., LTD., Saitama-shi, Saitama 331-0047 (JP); ONO, Atsushi, c/o Fujiyakuhin Co., LTD., Saitama-shi, Saitama 331-0047 (JP); SATO, Takahiro, c/o Fujiyakuhin Co., LTD., Saitama-shi, Saitama 331-0047 (JP)
(74) Representative: Hartz, Nikolai
(86) International application number: PCT/JP2004/010456
(87) International publication number: WO 2005/009991

(57) **Abstract**

Provided is a process for producing 1,2,4-triazole compound (5), or a salt or hydrate thereof which comprises reacting compound (1) with Rc-X (2) to give compound (3), reacting compound (3) with a nitrilization agent to give compound (4), and then removing the group Rc, as shown by the reaction scheme: 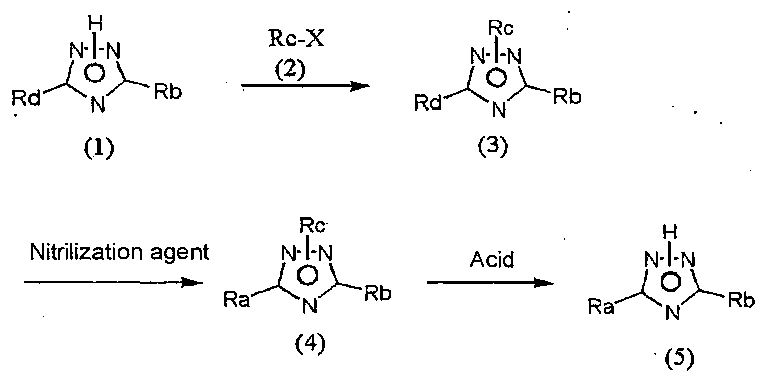 (Wherein Ra, Rb and Rd represent a group, Rc represents a group which can be removed by an acid)
A 1,2,4-triazole compound (5) having an optionally substituted 2-cyanopyridin-4-yl group at 3-position and an optionally substituted aromatic group at 5-position which inhibits a xanthine oxidase and is useful for treatment of gout and hyperuricemia can be obtained from compound (1) in a high yield without requiring isolation of reaction products in the course of reactions.

## Description

### Technical Field

The present invention relates to a process for producing novel 1,2,4-triazole compounds which have aromatic substituents at 3- and 5-positions, and intermediates thereof.

### Background Art

For the treatment of gout owing to hyperuricemia, it has been conducted to improve lifestyle habits after calming an attack by the treatment with a drug such as a steroidal or nonsteroidal antiinflammatory drug. However, in the case where gouty arthritis is repeated, gouty tophus is accompanied or a serum uric acid level is not less than 8 mg/dL even though the hyperuricemia is asymptomatic, a drug for lowering the serum uric acid level is applied, and a drug is administered depending on the clinical state of a patient (whether it is decreased uric acid excretion or increased uric acid production, whether there is a risk of urinary lithiasis, or the like). As a means to lower a serum uric acid level, drugs having a xanthine oxidase-inhibitory action have been used. However, only allopurinol has been clinically used among these drugs.

Under such circumstances, the present inventors have found that novel 1,2,4-triazole compounds having an optionally substituted 2-cyanopyridin-4-yl group at 3-position and an optionally substituted aromatic group at 5-position inhibit a xanthine oxidase and are useful for treatment of gout and hyperuricemia, and have previously filed a patent application (Patent Document 1). The compounds can be prepared according to a method shown by the following reaction scheme:

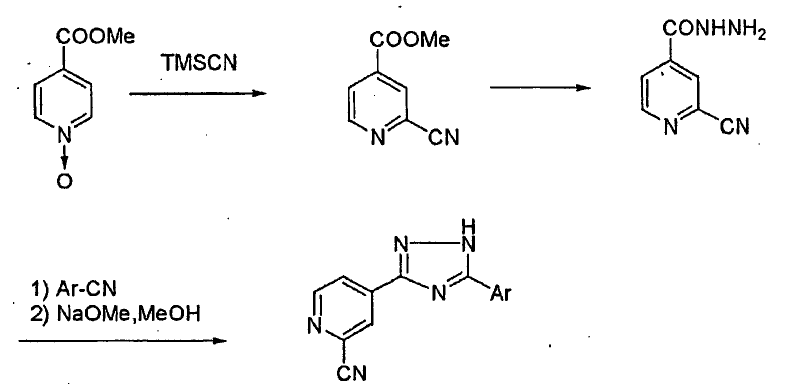

wherein TMS represents trimethylsilyl group and Ar represents an aromatic group.

Although this method can achieve the object in a small-scale production, there were such problems that the process for production of a substituted or unsubstituted 2-cyanoisonicotinic acid hydrazide is complicated, and a reaction solvent must be selected in compliance with the physical property of the product compound in each step, and isolation of a product is required in each step. Furthermore, the overall yield is not sufficiently high, and therefore there is a problem in the production on an industrial scale.
Patent Document 1: JP-A-2002-017825

### Disclosure of the Invention

### Problem to be solved by the Invention

An object of the present invention is to provide a process suitable for mass production of a 1,2,4-triazole compound which has an optionally substituted 2-cyanopyridin-4-yl group at 3-position and an optionally substituted aromatic group at 5-position and which is useful for treatment of gout and hyperuricemia, as well as intermediates thereof.

### Means to solve the Problem

The present inventors have found that a 1,2,4-triazole compound which has an optionally substituted 2-cyanopyridin-4-yl group at 3-position and an optionally substituted aromatic group at 5-position can be obtained in an extremely high yield without requiring isolation in each step by preliminarily preparing a 1,2,4-triazole compound having a pyridine N-oxide-4-yl group optionally substituted at a position other than 2-position at 3-position and an optionally substituted aromatic group at 5-position, converting the hydrogen atom bonded to the nitrogen atom of the triazole ring of this compound to a group which makes the resulting compound soluble in an organic solvent and which can be removed by an acid followed by nitrilizaton of the resulting compound, and removing said group, and have completed the invention.

The process of the present invention is represented by the following reaction scheme:

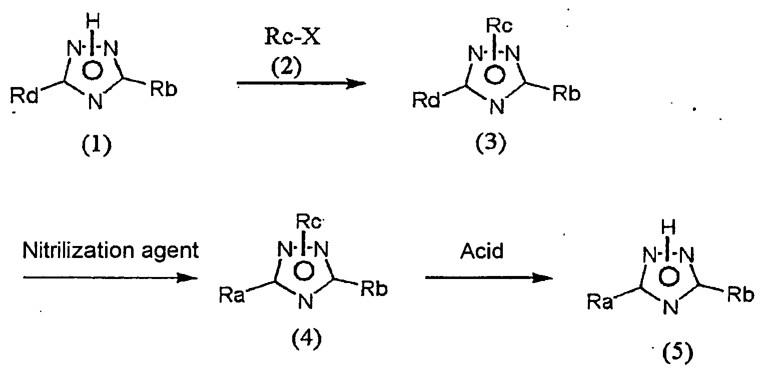

wherein Rd represents a pyridine N-oxide-4-yl group optionally substituted at a position other than 2-position, Rb represents an optionally substituted pyridyl or phenyl group, Rc represents a group which makes the compounds of formulae (3) and (4) soluble in an organic solvent and which can be removed by an acid and, in the compounds of formulae (3) and (4), Rc represents a group bonded to any of the nitrogen atoms contained in the triazole ring, Ra represents an optionally substituted 2-cyanopyridin-4-yl group, and X represents a halogen atom or sulfonate residue, the hydrogen atom in the compounds of formulae (1) and (5) being bonded to any of the nitrogen atom contained in the triazole ring.

Namely, the present invention provides a process for producing a compound represented by formula (3), and a salt or hydrate thereof, which comprises reacting a compound represented by formula (1) with a compound represented by formula (2).
The present invention also provides a process for producing a compound represented by formula (4), and a salt or hydrate thereof which comprises reacting a compound represented by formula (3) with a nitrilization agent.
Further, the present invention provides a process for producing a 1,2,4-triazole compound represented by formula (5), and a salt or hydrate thereof which comprises reacting a compound represented by formula (3) with a nitrilization agent to give a compound represented by formula (4), and then reacting the resulting compound with an acid.
In the process of the present invention, the compounds represented by formulae (3) and (4) are useful as an intermediate in the production.
Accordingly, the present invention provides 1,2,4-triazole compounds represented by formula (a):

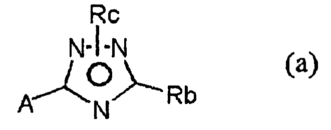

wherein A represents Rd or Ra, namely a pyridine N-oxide-4-yl group optionally substituted at a position other than 2-position or an optionally substituted 2-cyanopyridin-4-yl group, and Rb and Rc are as defined above and a salt or hydrate thereof.

### Effect of the Invention

A 1,2,4-triazole compound (5) having an optionally substituted 2-cyanopyridin-4-yl group at 3-position and an optionally substituted aromatic group at 5-position which inhibits a xanthine oxidase and is useful for treatment of gout and hyperuricemia can be obtained in an extremely high yield without requiring isolation of reaction products in the course of reactions.

### Best Mode for Carrying out the Invention

In the above scheme, the substituent which may substitute for the pyridine N-oxide-4-yl group represented by Rd and the 2-cyanopyridin-4-yl group represented by Ra include one to three groups selected from nitro group, halogen atoms, lower alkyl groups, lower alkoxy groups, lower alkylthio groups and phenyl group.

The substituent which may substitute for pyridyl or phenyl group represented by Rb includes one to four groups selected from cyano group, nitro group, halogen atoms, lower alkyl groups, substituted or unsubstituted lower alkoxy groups, lower alkylthio groups, N-lower alkylpiperadino groups, lower alkylamino groups, phenyl group and phenylthio group.

In Rd, Ra and Rb, the halogen atoms include fluorine, chlorine, bromine and iodine atoms. The lower alkyl groups include straight or branched alkyl groups having 1 to 6 carbon atoms, such as methyl, ethyl, isopropyl, and isobutyl groups. The substituted or unsubstituted lower alkoxy groups include substituted or unsubstituted straight or branched alkoxy groups having 1 to 6 carbon atoms, such as methoxy, ethoxy, isopropoxy, isobutoxy, methoxyethyloxy, methoxyethoxyethyloxy, and benzyloxymethyloxy groups. The lower alkylthio groups include straight or branched alkylthio groups having 1 to 6 carbon atoms, such as methylthio, ethylthio, and isopropylthio groups. The alkyl groups in the N-lower alkylpiperadino groups and the lower alkylamino groups include straight or branched alkyl groups having 1 to 6 carbon atoms.

As a group Rc, no limitation is imposed as long as it makes the compounds of formulae (3) and (4) soluble in an organic solvent and can be removed by an acid. Examples thereof include a group represented by formula (6)

-CH₂OR_{y}

(wherein Ry represents a substituted or unsubstituted alkyl group), diphenylmethyl group and p-alkoxybenzyl groups.

In formula (6), the substituted or unsubstituted alkyl group represented by Ry includes straight or branched alkyl groups having 1 to 6 carbon atoms (abbreviated as C₁₋₆ alkyl group), phenyl C₁₋₆ alkyl groups, C₁₋₆ alkoxy C₁₋₆ alkyl groups, and tri (C₁₋₆ alkyl) silyl-C₁₋₆ alkyl groups.
Examples of the C₁₋₆ alkyl and C₁₋₆ alkoxy groups include the same groups as mentioned above for the lower alkyl groups and the substituted or unsubstituted C1-C6 alkoxy groups such as methyl, ethyl, isopropyl, methoxy, ethoxy, and isopropoxy groups.
The p-alkoxybenzyl groups include p-C₁₋₆ alkoxybenzyl groups.
Particularly preferred examples of Rc include benzyloxymethyl, methoxymethyl, methoxyethoxymethyl, trimethylsilylethoxymethyl, diphenylmethyl, and p-methoxybenzyl groups.

A compound represented by formula (1) which is a starting material may be prepared by a method described in, for example, JP-A-47-7120, JP-A-61-152661A, JP-A-62-149673, JP-A-2002-528447, or European Patent Application No. 559363 specification. However, it is preferable to prepare compound (1) according to the following reaction scheme:

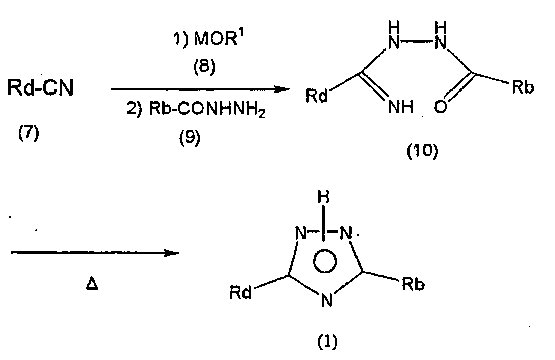

wherein M represents an alkali metal atom, R¹ represents a lower alkyl group, and Rb and Rd are as defined above.

Namely, a 4-cyanopyridine N-oxide (7) is reacted with an alkali metal alkoxide (8) and then with a hydrazide (9) to give compound (10), and subjection compound (10) to a ring closure reaction by heating to give a starting compound of formula (1).

In the reaction, the alkali metal alkoxide includes sodium methoxide. The reaction between the 4-cyanopyridine N-oxide (7) and the alkali metal alkoxide (8) is suitably effected in an alcohol at a temperature of from room temperature to 80°C for several hours. The reaction with the hydrazide (9) is suitably effected at a temperature of from 50 to 100°C for 30 minutes to several hours. It is sufficient that the subsequent ring-closure reaction is effected by heating to a temperature approximately from 100 to 150°C.

A series of reactions starting from a 4-cyanopyridine N-oxide (7) to a compound of formula (1) can be conducted in one pot without isolating intermediates and provides a starting compound (1) in a high yield.

The reaction between starting compound (1) and a compound of formula (2) is conducted in the presence of a base in an organic solvent. The organic solvent includes N,N-dimethylacetamide, N,N-dimethylformamide, N-methylpyrrolidone, acetonitrile, and dioxane. Among them, a polar solvent such as dimethylacetamide or dimethylformamide is preferred. The base is preferably an organic amine such as trimethylamine, triethylamine, diisopropylamine, or diisopropylethylamine. The reaction can be conducted at a temperature of from 50 to 150°C for 1 to 6 hours.

The nitrilization reaction of compound (3) is conducted in an organic solvent using a combination of a nitrilization agent such as trimethylsilylnitrile, potassium cyanide or sodium cyanide with the following reagent (provided that, when the nitrilization agent is potassium cyanide or sodium cyanide, a combination thereof with triethylamine or diisopropylamine is excluded): N,N-dimethyl carbamoyl chloride, 4-morpholinocarbonyl chloride, acetyl chloride, benzoyl chloride, p-toluenesulfonyl chloride, ethyl chlorocarbonate, methoxymethyl chloride, dimethyl sulfate, triethylamine, or diisopropylamine.
Among them, combinations of trimethylsilylnitrile with N,N-dimethylcarbamoyl chloride and trimethylsilylnitrile with triethylamine are preferred.

As the reaction solvent may be used N,N-dimethylacetamide, N,N-dimethylformamide, N-methylpyrrolidone, tetrahydrofuran, 1,4-dioxane, acetonitrile or dichloromethane, preferably N,N-dimethylacetamide.

With regard to the reaction temperature and reaction time, a nitrilization agent is added at 0 to 10°C, preferably not more than 5°C, and after stirring for 30 minutes to 2 hours, the reaction mixture is stirred at 20 to 70°C, preferably 30 to 60°C, for 5 to 20 hours, preferably 7 to 15 hours.

Then, the group on the nitrogen atom in the triazole ring of compound (4) obtained by nitrilization is removed using an acid to give 1,2,4-triazole compound (5).
As the acid to be used, any acid is usable as long as it can remove said group and does not decompose the present compounds. Examples of the preferred acids include organic acids such as p-toluenesulfonic acid and methanesulfonic acid, and inorganic acids such as hydrochloric acid and sulfuric acid.

As the reaction solvent may be used an alcohol such as methanol, ethanol, 2-propanol, or 2-butanol, or a mixed solvent of an alcohol and toluene. Ethanol or 2-propanol is preferred.
The reaction can be conducted with stirring at a temperature from 50 to 150°C, preferably 80 to 120°C, for 3 to 20 hours, preferably 5 to 15 hours.

The reactions from compound (1) to compound (5) can be conducted in an organic solvent in one pot without isolating intermediates to provide the product in a high yield, whereby the method is industrially advantageous.

Resulting compound (5) may be isolated as an acid addition salt of the acid used in the acid treatment in some cases. The acid addition salt may be converted to a free base of compound (5) by neutralization, if desired. The base used for neutralization of compound (5) varies depending on the acid to be neutralized and sodium hydrogen carbonate, potassium carbonate or the like can be preferably used. As the reaction solvent may be used alcohol-water solvents. Preferred alcohols include lower alcohols such as ethanol, 2-propanol and 2-butanol. The reaction temperature is suitably from 10 to 50°C, preferably 20 to 30°C.

In the 1,2,4-triazole compounds of the present invention having the same basic skeleton, the following isomers (A), (B) and (C) exist, which are all referred to as 1,2,4-triazole compounds represented by the following general formulae.

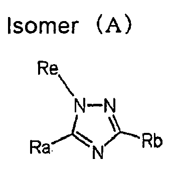

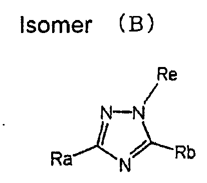

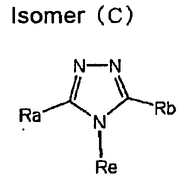

wherein Ra and Rb are as defined above, and Re represents a hydrogen atom or Rc.

### Examples

The present invention will be explained in detail by way of examples which are not intended to restrict the invention.

### Reference Example 1

### Preparation of 5-(1-oxy-4-pyridyl)-3-(4-pyridyl)-1,2,4-triazole

To a suspension of 4-cyanopyridine-N-oxide (130.0 g) in methanol (1040 mL) was added sodium methoxide (5.85 g) under an argon atmosphere, and the mixture was stirred at room temperature for 2 hours. Then isonicotinic acid hydrazide (148.43 g) was added thereto and the resulting mixture was refluxed for 1 hour. The reaction solution was cooled to room temperature, and N,N-dimethylformamide (DMF, 910 mL) was added thereto. After distilling away methanol, the resulting mixture was heated at an internal temperature of 105°C for 8 hours. After the reaction, the reaction mixture was cooled to room temperature. Precipitated yellow crystals were filtered, and then washed with DMF (130 mL) and methanol (130 mL×2). The resulting crystals were dried at 60°C for 15 hours under reduced pressure to give 234.32 g of the captioned compound as yellow crystals.

¹H―NMR(DMSO-d₆) δ(ppm) 7.98-8.03(m, 4H), 8.36(d, 2H, J=7.02Hz), 8.75-8.77(m,2H)

### Reference Example 2

### Preparation of 5-(1-oxy-4-pyridyl)-3-(2-methyl-4-pyridyl)-1,2,4-triazole

To a suspension of 4-cyanopyridine-N-oxide (0.99 g) in methanol (8.0 mL) was added sodium methoxide (0.045 g) under an argon atmosphere, and the mixture was stirred at room temperature for 2 hours. Then 2-methyl-isonicotinic acid hydrazide (1.25 g) was added thereto and the resulting mixture was refluxed for 1 hour. The reaction solution was cooled to room temperature, and N,N-dimethylformamide (DMF, 14.0 mL) was added thereto. After distilling away methanol, the resulting mixture was heated at an external temperature of 110°C for 23 hours. After the reaction, the reaction mixture was cooled to room temperature. Precipitated yellow crystals were filtered, and then washed with DMF (3.0 mL×6) and methanol (3.0 mL×3). The resulting crystals were dried at 80°C for 4.5 hours under reduced pressure to give 1.75 g of the captioned compound as yellow crystals.

¹H-NMR (DMSO- d₆) δ (ppm) : 2.58(s, 3H), 7.64(d, 1H, J=5.13Hz), 7.88(s, 1H), 7.99-8.03(m, 2H), 8.36(d, 2H, J=7.29Hz), 8.62(d, 1H, J=5.13Hz)

### Reference Example 3

### Preparation of 5-(1-oxy-4-pyridyl)-3-(2-chloro-4-pyridyl)-1,2,4-triazole

To a suspension of 4-cyanopyridine-N-oxide (5.00 g) in methanol (40.0 mL) was added sodium methoxide (0.22 g) in an argon atmosphere, and the mixture was stirred at room temperature for 2 hours. Then 2-chloro-isonicotinic acid hydrazide (7.14 g) was added thereto, and the resulting mixture was refluxed for 1.5 hours and DMF (35.0 mL) was added thereto. After distilling away methanol, the resulting mixture was heated to 120°C for 24 hours. After heating, the reaction mixture was cooled to room temperature. Precipitated white crystals were filtered, washed successively with DMF and methanol, and dried under reduced pressure to give 9.50 g of the captioned compound as white crystals.

¹H NMR(DMSO-d₆) δ(ppm) : 7.99-8.04 (m, 4H), 8.38 (d, 2H, J=1.35Hz), 8.59 (d, 1H, J=4.86Hz)

### Reference Example 4

### Preparation of 5-(1-oxy-4-pyridyl)-3-(2-phenyl-4-pyridyl)-1,2,4-triazole

To a suspension of 4-cyanopyridine-N-oxide (1.63 g) in methanol (13.0 mL) was added sodium methoxide (0.074 g) under an argon atmosphere, and the mixture was stirred at room temperature for 2 hours. Then 2-phenyl-isonicotinic acid hydrazide (2.90 g) was added thereto, and the resulting mixture was refluxed for 2 hours, and DMF (30.0 mL) was added thereto. After distilling away methanol, the resulting mixture was heated to 120°C for 13.5 hours.
After heating, the reaction mixture was cooled to room temperature. Precipitated white crystals were filtered, washed with methanol, and dried under reduced pressure to give 1.97 g of the captioned compound as yellow crystals.

¹H NMR(DMSO-d₆) δ (ppm) : 7.50-7.59 (m, 3H), 7.96 (d, 1H, J=5.13Hz), 8.05 (d, 2H, J=6.75Hz), 8.16 (d, 2H, J=7.56Hz), 8.37 (d, 2H, J=6.75Hz), 8.53 (s, 1H), 8.85 (d, 1H, J=5.13Hz)

### Reference Example 5

### Preparation of 5-(1-oxy-4-pyridyl)-3-[3-cyano-4-{2-(2-methoxyethoxy)ethoxy}phenyl]-1,2,4-triazole

To a suspension of 3-cyano-4-{2-(2-methoxyethoxy)ethoxy}benzonitrile (4.22 g) in methanol (34.0 mL) was added sodium methoxide (0.28 g) under an argon atmosphere, and the mixture was stirred at room temperature for 7.5 hours. Then isonicotinic acid hydrazide-N-oxide (2.62 g) was added thereto, and the resulting mixture was refluxed for 11 hours. After reflux, the reaction mixture was cooled to room temperature. Precipitated yellow crystals were filtered, washed with methanol, and dried under reduced pressure to give 2.73 g of the captioned compound as yellow crystals.

¹H NMR(DMSO-d₆) δ (ppm) : 3.26 (s, 3H), 3.46-3.49 (m, 2H), 3.63-3.66 (m, 2H), 3.83 (t, 2H, J=4.59Hz), 4.37 (t, 2H, J=4.59Hz), 7.48 (d, 1H, J=8.91Hz), 7.99 (dd, 2H, J=5.27, 1.62Hz), 8.28-8.35 (m, 4H)

### Example 1

### (1) Preparation of N-benzyloxymethyl-5-(1-oxy-4-pyridyl)-3-(4-pyridyl)-1,2,4-triazole

To a suspension of the yellow crystals (70.0 g) obtained in Reference Example 1 in N,N-dimethylacetoamide (700 mL) was added dropwise benzyl chloromethyl ether (46.6 mL) under an argon atmosphere, and the mixture was stirred at room temperature for 10 minutes. Triethylamine (46.9 mL) was added dropwise thereto, and the resulting reaction mixture was heated to an internal temperature of 85°C for 2 hours to give a solution of N-benzyloxymethyl-5-(1-oxy-4-pyridyl)-3-(4-pyridyl)-1,2,4-triazole. A part of the solution was separated to provide the following data. The captioned compound was white crystals (a mixture of isomers).

¹H-NMR (DMSO-d₆) δ (ppm) : 4.72(s, 2H), 4.73(s, 2H), 5.78(s, 2H), 5.82(s, 2H), 7.29-7.37(m, 10H), 7.90(dd, 2H, J=4.59, 1.62Hz), 7.94(dd, 2H, J=5.13, 1.89Hz), 7.99-8.01(m,4H), 8.33 (dd, 2H, J=5.13, 1.89Hz), 8.43(dd, 2H, J=4.59, 1.62Hz), 8.73-8.76(m,2H), 8.82-8.84(m,2H)

### (2) Preparation of N-benzyloxymethyl-5-(2-cyano-4-pyridyl)-3-(4-pyridyl)-1,2,4-triazole

The solution obtained in (1) was cooled to an internal temperature of 5°C, and trimethylsily cyanide (46.8 mL) was added dropwise. After stirring for 10 minutes, N,N-dimethylcarbamoyl chloride (64.7 mL) was added dropwise, and the reaction mixture was stirred at an internal temperature of 5°C for 1 hour. The internal temperature of the reaction mixture was gradually raised to 35°C and the reaction mixture was stirred for 10 hours. The reaction mixture was cooled to 5°C and 0.5 mol/L aqueous potassium carbonate solution (878 mL) was gradually added to make the reaction mixture basic. The internal temperature of the reaction mixture was brought to room temperature, toluene (350 mL) was added and the reaction mixture was stirred to dissolve precipitated products. This solution was extracted with toluene (1750 mL). An organic layer was washed with water (700 mL×2), and dried over magnesium sulfate (140.0 g). The toluene extract of the resulting N-benzyloxymethyl-5-(2-cyano-4-pyridyl)-3-(4-pyridyl)-1,2,4-triazole was concentrated to 700 mL and a part of this solution was isolated to provide the following data. The captioned compound was white crystal (a mixture of isomers).

¹H-NMR (DMSO-d₆) δ (ppm) : 4 . 71 (s, 2H), 4.74(s, 2H), 5.83 (s, 2H), 5.88 (s, 2H), 7.28-7.35 (m, 10H), 7.92(dd, 2H, J=4.59, 1.62Hz), 8.02(dd, 2H, J=4.59, 1.62Hz), 8.22(dd, 1H, J=5.13, 1.62Hz), 8.32(dd, 1H, J=5.13, 1.62Hz), 8.49(dd, 1H, J=1.62, 0.81Hz), 8.54(dd, 1H, J=1.62, 0.81Hz), 8.76(dd, 2H, J=4.59, 1.62Hz), 8.85(dd, 2H, J=4.59, 1.62Hz), 8.92(dd, 1H, J=5.13, 0.81Hz), 9.01(dd, 1H, J=5.13, 0.81Hz)

### Example 2

### Preparation of 5-(2-cyano-4-pyridyl)-3-(4-pyridyl)-1,2,4-triazole p-toluenesulfonate

To the toluene solution obtained in Example 1 (2) was added 2-propanol (700 mL), and the mixture was stirred. To the resulting solution was added p-toluenesulfonic acid monohydrate (151.16 g) and the resulting mixture was stirred for 8 hours at an internal temperature of 80°C. The mixture was brought to room temperature, and the precipitated crystals were taken out and washed with 2-propanol (210 mL×2). The white crystals were dried under reduced pressure at 60°C for 15 hours to give 106.0 g of the captioned compound as white crystals. Subsequently, 90.0 g of the crystals was suspended in a mixture of 2-butanol (49 mL) and water (491 mL) and heated to an internal temperature of 80°C for 1 hour. The internal temperature was brought to room temperature, and the crystals were filtered and washed with a mixture of 2-butanol and water (1:10) (270 mL×3). The resulting crystals were dried under reduced pressure at 60°C for 15 hours to give 75.7 g of the captioned compound in a high purity.

¹H―NMR(DMSO-d₆)δppm:2.29(s,3H), 7.11 (m,2H), 7.48 (dd, 2H, J=6.48, 1.62Hz) , 8.32-8.35(m, 3H) , 8.57(dd, 1H, J=1.62, 0.81Hz) , 8.94-8.98(m, 3H)

### Example 3

### Preparation of 5-(2-cyano-4-pyridyl)-3-(4-pyridyl)-1,2,4-triazole

To the white crystals (50.5g) obtained in Example 2 was added 2-propanol (937.5 mL) and water (312.5 mL), and the resulting mixture was heated and dissolved at an internal temperature of 80°C. Immediately thereafter, the solution was filtered and the filtrate was cooled to an internal temperature of 20°C. To the resulting suspension was added dropwise 0.52 mol/l of an aqueous sodium hydrogen carbonate solution (250 mL), and the mixture was stirred at room temperature for 2 hours. Then the crystals were filtered and washed with water (150 mL×3) and 2-butanol (150 mL×2). The crystals were dried under reduced pressure at 80°C for 15 hours to give 29.4 g of the captioned compound as pale yellow crystals.

¹H―NMR(DMSO-d₆)δppm:8.02(dd, 2H, J=4.59, 1.62Hz),8.32(dd, 1H, J=5.13, 1.62Hz), 8.55(dd, 1H, J=1.62, 1.08Hz), 8.80(dd, 2H, J=4.59, 1.62Hz), 8.93 (dd, 1H, J=5.13, 1.08Hz)

### Example 4

### (1) Preparation of N-methoxymethyl-5-(1-oxy-4-pyridyl)-3-(4-pyridyl)-1,2,4-triazole

To a suspension of the yellow crystals (34.7 g) obtained in Reference Example 1 in N,N-dimethylacetamide (347 mL) was added dropwise chloromethyl methyl ether (13.7 mL) under an argon atmosphere, and the mixture was stirred at room temperature for 10 minutes. Triethylamine (25.3 mL) was added dropwise and the resulting mixture was heated to an internal temperature of 83°C for 2 hours. A part of the reaction mixture was brought to room temperature, and purified by column chromatography on silica gel (chloroform: methanol=20:1) to confirm the captioned compound as white crystals (a mixture of isomers).
¹H-NMR(DMSO-d₆)δ(ppm) : 3.43 (s, 3H), 3.44(s, 3H), 5.67(s, 2H), 5.70(s,2H), 7.89(dd, 2H, J=4.32, 1.89Hz), 7.93(dd, 2H, J=5.13, 2.16Hz), 7.99(dd, 4H,J=4.59, 1.62Hz), 8.32(dd, 2H, J=5.13, 2.16Hz), 8.43(dd, 2H, J=4.32, 1.89Hz),8.74(dd, 2H, J=4.59, 1.62Hz), 8.84(dd, 2H, J=4.59, 1.62Hz)

### (2) Preparation of N-methoxymethyl-5-(2-cyano-4-pyridyl)-3-(4-pyridyl)-1,2,4-triazole

The solution obtained in (1) was cooled to an internal temperature of 15°C, and trimethylsilyl cyanide (23.8 mL) was added dropwise. After stirring for 10 minutes, N,N-dimethylcarbamoyl chloride (26.7 mL) was added dropwise, and the reaction mixture was stirred at room temperature for 1 hour and at an internal temperature of 57°C for 3 hours. The reaction mixture was cooled in an ice bath and 0.5 mol/L aqueous potassium carbonate solution (581 mL) was gradually added to make the reaction mixture basic. This solution was extracted with toluene (1042 mL) and the aqueous layer was further extracted with toluene (347 mL). The toluene extracts were combined, washed with water (347 mL) and saturated brine (347 mL), and dried over magnesium sulfate (69.5 g). The toluene extract was concentrated to 347 mL after passing through a filter to remove the magnesium sulfate. A part of the solution was isolated to confirm the captioned compound. The captioned compound was white crystal (a mixture of isomers).

¹H-NMR(DMSO-d₆) δ (ppm) : 3. 42 (s, 3H), 3.44 (s, 3H), 5.71(s, 2H), 5.75 (s, 2H), 7.91(dd, 2H, J=4.59, 1.62Hz), 8.02(dd, 2H, J=4.32, 1.62Hz), 8.23(dd, 1H, J=5.13, 1.62Hz), 8.32(dd, 1H, J=5.13, 1.62Hz), 8.48(dd, 1H, J=1.62, 0.81Hz), 8.53(dd, 1H, J=1.62, 0.81Hz), 8.76(dd, 2H, J=4.59, 1.62Hz),8.86(dd, 2H, J=4.32, 1.62Hz), 8.92(dd, 1H, J=5.13, 0.81Hz), 9.02(dd, 1H, J=5.13, 0.81Hz)

### Example 5

### (1) Preparation of N-(2-methoxyethoxymethyl)-5-(1-oxy-4-pyridyl)-3-(4-pyridyl)-1,2,4-triazole

To a suspension of the yellow crystals (1.00 g) obtained in Reference Example 1 in N,N-dimethylacetamide (10.0 mL) was added dropwise 2-methoxyethoxymethylchloride (0.55 mL), and the mixture was stirred at room temperature for 10 minutes. Then diisopropylethylamine (0.82 mL) was added dropwise and the resulting mixture was heated to an external temperature of 90°C for 2 hours. A part of the reaction mixture was brought to room temperature, and purified by column chromatography on silica gel (chloroform: methanol=20:1) to confirm the captioned compound as white crystals (a mixture of isomers).

¹H-NMR (DMSO- d₆) δ(ppm) :3.19(s, 3H), 3.20(s, 3H), 3.46-3.49(m, 4H), 3.76-3.80(m,4H), 5.73(s,2H), 5.76(s,2H), 7.89-7.92(m,2H),7.94(d,2H, J=7.02Hz), 7.99(dd, 4H, J=5.13, 1.62Hz), 8.31-8.33(m,2H) 8.44 (d, 2H, J=7.02Hz), 8.74(dd, 2H, J=5.13, 1.62Hz), 8.85(dd, 2H, J=5.13 1.62Hz)

### (2) Preparation of N-(2-methoxyethoxymethyl)-5-(2-cyano-4-pyridyl)-3-(4-pyridyl)-1,2,4-triazole

The reaction mixture obtained in (1) was cooled in an ice bath for 25 minutes, and trimethylsilyl cyanide (0.67 mL) was added dropwise. After stirring for 10 minutes, N,N-dimethylcarbamoyl chloride (0.93 mL) was added dropwise, and the mixture was stirred in an ice bath for 1 hour and, after raising the external temperature to 60°C, it was stirred for 3 hours. The reaction mixture was cooled in an ice bath and 0.5 mol/L aqueous potassium carbonate solution (12.0 mL) was gradually added to make the reaction mixture basic. This solution was extracted with toluene (30.0 mL), and the extract was washed with water (10.0 mL×2) and dried over magnesium sulfate. The solvent was distilled away under reduced pressure and the resulting residue was purified by column chromatography on silica gel (acetone:chloroform=1:10→1:5) to give 1.34 g of the captioned compound as a colorless oil (a mixture of isomers).

¹H―NMR(DMSO-d₆) δ(ppm) : 3.19(s, 3H), 3.20(s, 3H), 3.33-3.49(m, 4H),3 .75-3.81(m, 4H), 5.77(s, 2H), 5.82(s, 2H), 7.93(dd, 2H, J=4.59, 1.62Hz),8.01(dd, 2H, J=4.32, 1.62Hz), 8.24(dd, 1H, J=5.13, 1.62Hz), 8.30-8.32(m,1H), 8.50-8.51(m, 1H), 8.53(dd, 1H, J=1.62, 0.81Hz), 8.75(dd, 2H, J=4.32, 1.62Hz), 8.86(dd, 2H, J=4.59, 1.62Hz),8.91(dd, 1H, J=5.13, 0.81Hz), 9.02(dd, 1H, J=5.13, 0.81Hz)

### Example 6

### (1) Preparation of N-[2-(trimethylsilyl)ethoxymethyl-5-(1-oxy-4-pyridyl)-3-(4-pyridyl)-1,2,4-triazole

To a suspension of the yellow crystals (1.00 g) obtained in Reference Example 1 in N,N-dimethylacetamide (10.0 mL) was added dropwise 2-(chloromethoxy)ethyltrimethylsilane (0.85 mL) under an argon atmosphere, and the mixture was stirred at room temperature for 10 minutes. Diisopropylethylamine (0.82 mL) was added dropwise and the resulting mixture was heated to 90°C for 2 hours. A part of the reaction mixture was brought to room temperature, and purified by column chromatography on silica gel (chloroform: methanol=20:1) to confirm the captioned compound as a colorless oil (a mixture of isomers).

¹H―NMR(DMSO-d₆) δ (ppm): -0.01 (s, 9H), 0.00(s, 9H), 0.89-1.00(m, 4H),3.76-3.84(m, 4H), 5.77(s, 2H), 5.81(s, 2H), 7.96-7.98(m,2H), 8.00-8.07(m, 6H), 8.38-8.41(m,2H), 8.49-8.52(m,2H), 8.79-8.81(m,2H), 8.90 (dd, 2H, J=4,59, 1.62Hz)

### (2) Preparation of N-[2-(trimethylsilyl)ethoxymethyl)-5-(2-cyano-4-pyridyl)-3-(4-pyridyl)-1,2,4-triazole

The reaction mixture obtained in (1) was cooled in an ice bath for 35 minutes, and trimethylsilyl cyanide (0.67 mL) was added dropwise. After stirring for 10 minutes, N,N-dimethylcarbamoyl chloride (0.92 mL) was added dropwise, and the mixture was stirred for 1 hour. After raising the external temperature to 60°C, it was stirred for 3 hours. The reaction mixture was cooled in an ice bath and 0.5 mol/L aqueous potassium carbonate solution (12.0 mL) was gradually added to make the reaction mixture basic. This solution was extracted with toluene (30.0 mL), and the extract was washed with water (10.0 mL×2) and dried over magnesium sulfate. The solvent was distilled away under reduced pressure and the resulting residue was purified by column chromatography on silica gel (acetone:chloroform=1:10) to give 1.5 g of the captioned compound as white crystals (a mixture of isomers).

¹H-NMR (DMSO- d₆) δ (ppm) : 0.00 (s, 18H), 0.95 (t, 4H, J=7.97Hz), 3.76-3.83(m, 4H), 5.82(s, 2H), 5.87(s, 2H), 7.99-8.01(m,2H), 8.07(dd, 2H, J=4.32, 1.62Hz), 8.32(dd, 1H, J=5.13, 1.89Hz), 8.37(dd, 1H, J=5.13, 1.62Hz), 8.57(dd, 1H, J=1.89, 0.81Hz), 8.59(dd, 1H, J=1.62, 0.81Hz), 8.81-8.83(m,2H), 8.91-8.93(m,2H), 8.98(dd, 1H, J=5.13, 0.81Hz), 9.09(dd, 1H, J=5.13,0.81Hz)

### Example 7

### Preparation of 5-(2-cyano-4-pyridyl)-3-(4-pyridyl)-1,2,4-triazole hydrochloride

To the compound (1.00 g) isolated from the solution obtained in Example 1 (2) were added toluene (6.5 mL) and 2-propanol (6.5 mL), and the mixture was stirred. To the resulting solution was added conc. hydrochloric acid (0.75 mL) and the resulting mixture was stirred for 5 hours at an external temperature of 90°C. The mixture was brought to room temperature, and the precipitated crystals were taken out and washed with 2-propanol (3.0 mL×3). The crystals were dried under reduced pressure at 80°C for 3 hours to give 0.73 g of the captioned compound as pale gray crystals.

¹H-NMR(DMSO-d₆)δ(ppm) : 8.35-8.38(m,3H), 8.57(s, 1H), 8.9 4-8.97(m, 3H)

### Example 8

### Preparation of 5-(2-cyano-4-pyridyl)-3-(4-pyridyl)-1,2,4-triazole sulfate

To the compound (1.0 g) isolated from the solution obtained in Example 1 (2) were added toluene (6.5 mL) and 2-propanol (6.5 mL), and the mixture was stirred. To the resulting solution was added conc. sulfuric acid (0.43 mL) and the resulting mixture was stirred for 6 hours at an external temperature of 90°C. The mixture was brought to room temperature, and the precipitated crystals were taken out and washed with 2-propanol (3.0 mL×3). The crystals were dried under reduced pressure at 80°C for 4 hours to give 0.81 g of the captioned compound as white crystals.

¹H-NMR(DMSO-d6)δ(ppm) : 8.32-8.35(m,3H), 8.57(dd, 1H, J=1.76, 0.95Hz), 8.94-8.98 (m,3H)

### Example 9

### Preparation of 5-(2-cyano-4-pyridyl)-3-(4-pyridyl)-1,2,4-triazole methanesulfonate

To the compound (1.0 g) isolated from the solution obtained in Example 1 (2) were added toluene (6.5 mL) and 2-propanol (6.5 mL), and the mixture was stirred. To the resulting solution was added methanesulfonic acid (0.75 mL) and the resulting mixture was stirred for 6 hours at an external temperature of 90°C. The mixture was brought to room temperature, and the precipitated crystals were taken out and washed with 2-propanol (3.0 mL×3). The crystals were dried under reduced pressure at 80°C for 4 hours to give 0.84 g of the captioned compound as white crystals.

¹H-NMR (DMSO-d₆)δ(ppm) 2.34(s,3H), 8.32-8.35 (m,3H), 8.58 (dd,1H, J=1.62, 0.81Hz), 8.96-8.98(m, 3H)

### Example 10

### Preparation of N-(4-methoxybenzyl)-5-(2-cyano-4-pyridyl)-3-(4-pyridyl)-1,2,4-triazole

To a suspension of the yellow crystals (1.02 g) obtained in Reference Example 1 in N,N-dimethylacetamide (10.0 mL) was added dropwise 4-methoxybenzyl chloride (0.67 mL) under an argon atmosphere, and the mixture was stirred at room temperature for 10 minutes. Diisopropylethylamine (0.83 mL) was added dropwise and the resulting mixture was heated to an external temperature of 90°C for 5 hours and 20 minutes. After allowing the reaction mixture to stand overnight at room temperature, trimethylsilyl cyanide (0.68 mL) was added dropwise and the resulting mixture was stirred for 10 minutes. N,N-dimethylcarbamoyl chloride (0.94 mL) was added dropwise and the reaction mixture was stirred for 4 hours and 45 minutes after raising the external temperature to 60°C. The reaction mixture was cooled in an ice bath and 0.5 mol/L aqueous potassium carbonate solution (15.0 mL) was gradually added to make the reaction mixture basic. This solution was extracted with toluene (30 mL×2), and the extract was washed with water (15 mL×2) and dried over magnesium sulfate. The solvent was distilled away under reduced pressure, and the resulting residue was purified by column chromatography on silica gel (methanol:chloroform=1:20) and dried under reduced pressure at room temperature to give 0.31 g of the captioned compound as an oil (a mixture of isomers).

¹H-NMR (DMSO- d₆) δ(ppm) : 3.71(s, 6H), 5.63(s, 2H), 5.66(s, 2H), 6.86-6.91(m, 4H), 7.12-7.16(m, 4H), 7.77-7.79(m, 2H), 7.96-7.98 (m, 2H), 8.08-8.10(m, 1H), 8.28(dd, 1H, J=5.13, 1.62Hz), 8.41-8.42(m, 1H), 8.49 (dd, 1H, J=1.62, 0.81Hz), 8.71-8.74(m, 2H), 8.79-8.82(m, 2H), 8.89(dd, 1H, J=5.13, 0.81Hz), 8.95(dd, 1H, J=5.13, 0.81Hz)

### Example 11

### Preparation of N-benzhydryl-5-(2-cyano-4-pyridyl)-3-(4-pyridyl)-1,2,4-triazole

To a suspension of the yellow crystals (1.00 g) obtained in Reference Example 1 in N,N-dimethylacetamide (10.0 mL) was added dropwise benzhydryl chloride (0.85 mL) under an argon atmosphere, and the mixture was stirred at room temperature for 10 minutes. Diisopropylethylamine (0.67 mL) was added dropwise and the resulting mixture was heated to an external temperature of 90°C for 5 hours and 20 minutes. After allowing the reaction mixture to stand overnight at room temperature, trimethylsilyl cyanide (0.67 mL) was added dropwise and the resulting mixture was stirred for 10 minutes. N,N-dimethylcarbamoyl chloride (0.92 mL) was added dropwise and the reaction mixture was stirred for 3 hours after raising the external temperature to 60°C. The reaction mixture was cooled in an ice bath and 0.5 mol/L aqueous potassium carbonate solution (15.0 mL) was gradually added to make the reaction mixture basic. This solution was extracted with toluene (30 mL×2), and the extract was washed with water (15 mL×2) and dried over magnesium sulfate. The solvent was distilled away under reduced pressure, and the resulting residue was purified by column chromatography on silica gel (methanol:chloroform=1:30) and dried under reduced pressure at room temperature to give 0.21 g of the captioned compound as an oil (a mixture of isomers).

¹H-NMR(DMSO-d₆) δ(ppm): 7.29-7.42 (m, 22H), 7.67(dd, 2H, J=4.32, 1.62Hz), 7.92(dd, 2H, J=4.32, 1.62Hz), 7.94-7.96(m, 1H), 8.23(dd, 1H, J=5.13,1.62Hz), 8.28-8.29(m, 1H), 8.45-8.46(m, 1H), 8.70(dd, 2H, J=4.32, 1.62Hz), 8.82(dd, 2H, J=4.32, 1.62Hz), 8.86(dd, 1H, J=5.13, 0.54Hz), 8.94-8.96(m, 1H)

### Example 12

### Preparation of N-benzyloxymethyl-5-(2-cyano-4-pyridyl)-3-(2-methyl-4-pyridyl)-1,2,4-triazole

To a suspension of the yellow crystals (0.50 g) obtained in Reference Example 2 in N,N-dimethylacetamide (5.0 mL) was added dropwise benzyl chloromethyl ether (0.32 mL) under an argon atmosphere, and the mixture was stirred at room temperature for 10 minutes. Triethylamine (0.32 mL) was added dropwise and the resulting mixture was heated to an external temperature of 90°C for 3 hours. The reaction mixture was cooled in an ice bath and trimethylsilyl cyanide (0.32 mL) was added dropwise and the resulting mixture was stirred for 10 minutes. N,N-dimethylcarbamoyl chloride (0.44 mL) was added dropwise and the reaction mixture was stirred for 1 hour and then for 3 hours after raising the external temperature to 60°C. The reaction mixture was cooled in an ice bath and 0.5 mol/L aqueous potassium carbonate solution (6.0 mL) was gradually added to make the reaction mixture basic. This solution was extracted with toluene (15.0 mL), and the extract was washed with water (5.0 mL×2) and dried over magnesium sulfate. The solvent was distilled away under reduced pressure, and the resulting residue was purified by column chromatography on silica gel (acetone:chloroform=1:30) to give 0.63 g of the captioned compound as a colorless oil (a mixture of isomers).

¹H-NMR(DMSO-d₆) δ (ppm) : 2.58 (s, 3H), 2.59 (s, 3H), 4.71 (s, 2H), 4.73(s, 2H), 5.81(s, 2H), 5.87(s, 2H), 7.29-7.34 (m, 10H), 7.70-7.72(m, 1H), 7.76-7.77(m, 1H), 7.82-7.83(m, 1H), 7.89-7.90 (m, 1H), 8.22(dd, 1H, J=5.13, 1.62Hz), 8.31(dd, 1H, J=5.13, 1.62Hz), 8.48(dd, 1H, J=1.62, 0.81Hz), 8.53(dd, 1H, J=1.62, 0.81Hz), 8.62(dd, 1H, J=5.27, 0.81Hz), 8.69-8.71(m, 1H), 8.92(dd, 1H, J=5.13, 0.81Hz), 9.01(dd, 1H, J=5.13, 0.81Hz)

### Example 13

### Preparation of 5-(2-cyano-4-pyridyl)-3-(2-methyl-4-pyridyl)-1,2,4-triazole p-toluenesulfonate

To N-benzyloxymethyl-5-(2-cyano-4-pyridyl)-3-(2-methyl-4-pyridyl)-1,2,4-triazole (0.63 g) obtained in Example 12 were added 2-propanol (4.1 mL) and toluene (4.1 mL), and the mixture was stirred. To the resulting solution was added p-toluenesulfonic acid monohydrate (0.85 g), and the resulting mixture was stirred for 13 hours at an external temperature of 80°C. The mixture was brought to room temperature, and the precipitated crystals were taken out and washed with 2-propanol (3.8 mL×2). The white crystals were dried under reduced pressure at 60°C for 5 hours to give 0.58 g of the captioned compound as white crystals.

¹H-NMR (DMSO-d₆) δppm: 2.29(s, 3H), 2.77 (s, 3H), 7.11 (dd, 2H, J=7.83, 0.54Hz), 7.48(dd, 2H, J=7.83, 0.54Hz), 8.28-8.34(m, 3H), 8.57-8.58(m, 1H), 8.89(dd, 1H, J=5.94, 1.35Hz), 8.99(d, 1H, J=5.13Hz)

### Example 14

### (1) Preparation of N-benzyloxymethyl-5-(1-oxy-4-pyridyl)-3-(2-chloro-4-pyridyl)-1,2,4-triazole

To a suspension of 5-(1-oxy-4-pyridyl)-3-(2-chloro-4-pyridyl)-1,2,4-triazole (1.00 g) obtained in Reference Example 3 in N,N-dimethylacetamide (10.0 mL) was added dropwise benzyl chloromethyl ether (0.78 mL) under an argon atmosphere, and the mixture was stirred at room temperature for 10 minutes. Triethylamine (0.78 mL) was added dropwise, and the resulting mixture was heated to an external temperature of 120°C for 2 hours to give a solution of N-benzyloxymethyl-5-(1-oxy-4-pyridyl)-3-(2-chloro-4-pyridyl)-1,2,4-triazole. A part of the reaction mixture was brought to room temperature and purified by column chromatography on silica gel (methanol:chloroform = 1:20) to confirm the captioned compound as white crystals (a mixture of isomers).

¹H NMR(DMSO-d₆) δ (ppm) : 4.72 (s, 2H), 4.74 (s, 2H), 5.81 (s, 2H), 5.83(s, 2H), 7.27-7.36 (m, 10H), 7.90-7.96 (m, 3H), 7.99-8.02 (m, 5H), 8.33 (d, 2H, J=7.29Hz), 8.43 (d, 2H, J=7.29Hz), 8.59 (d, 1H, J=5.13Hz), 8.67 (d, 1H, J=5.13Hz)

### (2) Preparation of N-benzyloxymethyl-5-(2-cyano-4-pyridyl)-3-(2-chloro-4-pyridyl)-1,2,4-triazole

The solution of N-benzyloxymethyl-5-(1-oxy-4-pyridyl)-3-(2-chloro-4-pyridyl)-1,2,4-triazole obtained in (1) was cooled in an ice bath, and trimethylsilyl cyanide (0.58 mL) was added dropwise. After stirring for 10 minutes, N,N-dimethylcarbamoyl chloride (0.80 mL) was added dropwise, and the mixture was stirred in an ice bath for 1 hour and, after raising the external temperature to 60°C, it was stirred for 3 hours. The reaction mixture was cooled in an ice bath and 0.5 mol/L aqueous potassium carbonate solution (14.5 mL) was gradually added to make the reaction mixture basic. This solution was extracted twice with toluene, and the toluene extracts were combined, washed with water (15 mL×3) and dried over magnesium sulfate. The solvent was then distilled away under reduced pressure, and the resulting residue was purified by column chromatography on silica gel (methanol:chloroform = 1:50) to give 1.48 g of the captioned compound as white crystals (a mixture of isomers).

¹H NMR (DMSO-d₆) δ (ppm) : 4.72 (s, 2H), 4.73 (s, 2H), 5.86 (s, 2H), 5.89(s, 2H), 7.31-7.35 (m, 10H), 7.94 (d, 1H, J=5.13Hz), 8.02-8.04 (m, 3H), 8.23 (dd, 1H, J=5.13, 0.81Hz), 8.32 (dd, 1H, J=5.13, 0.81Hz), 8.50 (d, 1H, J=0.81Hz), 8.55 (d, 1H, J=0.81Hz), 8.61 (d, 1H, J=5.13Hz), 8.70 (d, 1H, J=5.13Hz), 8.93 (d, 1H, J=5.13Hz), 9.02 (d, 1H, J=5.13Hz)

### Example 15

### (1) Preparation of N-benzyloxymethyl-5-(1-oxy-4-pyridyl)-3-(2-phenyl-4-pyridyl)-1,2,4-triazole

To a suspension of 5-(1-oxy-4-pyridyl)-3-(2-phenyl-4-pyridyl)-1,2,4-triazole (0.90 g) obtained in Reference Example 4 in N,N-dimethylacetamide (9.0 mL) was added dropwise benzyl chloromethyl ether (0.92 mL) under an argon atmosphere, and the mixture was stirred at room temperature for 10 minutes. Triethylamine (0.92 mL) was added dropwise and the resulting mixture was heated to an external temperature of 90°C for 5.5 hours to give a solution of N-benzyloxymethyl-5-(1-oxy-4-pyridyl)-3-(2-phenyl-4-pyridyl)-1,2,4-triazole. After adding water, this reaction solution was extracted three times with toluene, and the toluene extracts were combined, washed twice with water and dried over magnesium sulfate. The solvent was then distilled away under reduced pressure, and the resulting residue was purified by thin layer chromatography on silica gel (methanol:chloroform=1:10) to confirm the captioned compound as white crystals (a mixture of isomers).

¹H NMR(DMSO-d₆) δ(ppm) : 4.76 (s, 4H), 5.85 (s, 2H), 5.86(s, 2H), 7.28-7.36 (m, 10H), 7.47-7.59 (m, 6H), 7.84 (d, 1H, J=5.13Hz), 7.96-7.99 (m, 3H), 8.03 (d, 2H, J=6.48Hz), 8.09-8.17 (m, 4H), 8.35 (d, 2H, J=6.48Hz), 8.43 (s, 2H), 8.46 (d, 2H, J=6.48Hz), 8.84 (d, 1H, J=5.13Hz), 8.91 (d, 1H, J=5.13Hz)

### (2) Preparation of N-benzyloxymethyl-5-(2-cyano-4-pyridyl)-3-(2-phenyl-4-pyridyl)-1,2,4-triazole

The solution of N-benzyloxymethyl-5-(1-oxy-4-pyridyl)-3-(2-phenyl-4-pyridyl)-1,2,4-triazole obtained in (1) was cooled in an ice bath, and trimethylsilyl cyanide (0.46 mL) was added dropwise. After stirring for 10 minutes, N,N-dimethylcarbamoyl chloride (0.63 mL) was added dropwise, and the mixture was stirred in an ice bath for 1 hour and, after raising the external temperature to 60°C, it was stirred for 3 hours. The reaction mixture was cooled in an ice bath and 0.5 mol/L aqueous potassium carbonate solution (8.6 mL) was gradually added to make the reaction mixture basic. This solution was extracted three times with toluene, and the extracts were dried over magnesium sulfate. The solvent was then distilled away under reduced pressure, and the resulting residue was purified by column chromatography on silica gel (methanol:chloroform=1:40) to give 1.27 g of the captioned compound as white crystals (a mixture of isomers).

¹H NMR (DMSO-d₆) δ (ppm) : 4.59 (s, 4H), 5.89 (s, 2H), 5.91(s, 2H), 7.30-7.36 (m, 10H), 7.50-7.59 (m, 6H), 7.86 (dd, 1H, J=5.13, 1.35Hz), 7.99 (dd, 1H, J=5.13, 1.35Hz), 8.10-8.18 (m, 4H), 8.26 (dd, 1H, J=5.13, 1.62Hz), 8.35 (dd, 1H, J=5.13, 1.62Hz), 8.46 (d, 1H, J=1.35Hz), 8.50 (d, 1H, J=1.35Hz), 8.53 (dd, 1H, J=1.62, 0.81Hz), 8.58 (dd, 1H, J=1.62, 0.81Hz), 8.86 (dd, 1H, J=5.13, 0.81Hz), 8.92-8.94 (m, 2H), 9.02 (dd, 1H, J=5.13, 0.81Hz)

### Example 16

### (1) Preparation of N-benzyloxymethyl-5-(1-oxy-4-pyridyl)-3-[3-cyano-4-{2-(2-methoxyethoxy)ethoxy}phenyl]-1,2,4-triazole

To a suspension of 5-(1-oxy-4-pyridyl)-3-[3-cyano-4-{2-(2-methoxyethoxy)ethoxy}phenyl]-1,2,4-triazole (1.00 g) obtained in Reference Example 5 in N,N-dimethylacetamide (10.0 mL) was added dropwise benzyl chloromethyl ether (1.26 mL) under an argon atmosphere, and the mixture was stirred at room temperature for 10 minutes. Triethylamine (1.26 mL) was added dropwise and the resulting mixture was heated to an external temperature of 90°C for 6.5 hours to give a solution of N-benzyloxymethyl-5-(1-oxy-4-pyridyl)-3-[3-cyano-4-{2-(2-methoxyethoxy)ethoxy}phenyl]-1,2,4-triazole. After adding water, this reaction solution was extracted twice with chloroform, and the chloroform extracts were combined, washed with water and dried over magnesium sulfate. The solvent was then distilled away under reduced pressure, and the resulting residue was purified by thin layer chromatography on silica gel (methanol:chloroform=1:10) to confirm the captioned compound as yellow crystals (a mixture of isomers).

¹H NMR(DMSO-d₆) δ (ppm) : 3.26 (s, 6H), 3.46-3.50 (m, 4H), 3.63-3.67(m, 4H), 3.83-3.86 (m, 4H), 4.34-4.41(m, 4H), 7.29-7.34 (m, 10H), 7.44 (d, 1H, J=8.91Hz), 7.51 (d, 1H, J=8.91Hz), 7.93 (d, 2H, J=6.75Hz), 7.99 (d, 2H, J=6.75Hz), 8.16 (dd, 1H, J=8.91, 1.89Hz), 8.23 (d, 1H, J=1.89Hz), 8.29-8.33 (m, 4H), 8.42 (d, 2H, J=6.75Hz)

### (2) Preparation of N-benzyloxymethyl-5-(2-cyano-4-pyridyl)-3-[3-cyano-4-{2-(2-methoxyethoxy)ethoxy}phenyl]-1,2,4-triazole

The solution of N-benzyloxymethyl-5-(1-oxy-4-pyridyl)-3-[3-cyano-4-{2-(2-methoxyethoxy)ethoxy}phenyl]-1,2,4-triazole obtained in (1) was cooled in an ice bath, and trimethylsilyl cyanide (0.42 mL) was added dropwise. After stirring for 10 minutes, N,N-dimethylcarbamoyl chloride (0.58 mL) was added dropwise, and the mixture was stirred in an ice bath for 1 hour and, after raising the external temperature to 60°C, it was stirred for 3 hours. The reaction mixture was cooled in an ice bath and 0.5 mol/L aqueous potassium carbonate solution (7.9 mL) was gradually added to make the reaction mixture basic. This solution was extracted five times with toluene, and the extracts were dried over magnesium sulfate. The solvent was then distilled away under reduced pressure, and the resulting residue was purified by column chromatography on silica gel (methanol:chloroform=1:40) to give 1.12 g of the captioned compound as yellow crystals (a mixture of isomers).

¹H NMR(DMSO-d₆) δ (ppm) : 3.25 (s, 3H), 3.26 (s, 3H), 3.46-3.49 (m, 4H), 3.63-3.67(m, 4H), 3.83-3.85(m, 4H), 4.37-4.41(m, 4H), 7.32-7.33 (m, 10H), 7.46 (d, 1H, J=9.18Hz), 7.53 (d, 1H, J=9.18Hz), 8.16-8.34 (m, 6H), 8.49 (s, 1H), 8.53 (s, 1H), 8.91 (d, 1H, J=4.86Hz), 9.00 (d, 1H, J=4.86Hz)

### Example 17

### Preparation of 5-(2-cyano-4-pyridyl)-3-(2-chloro-4-pyridyl)-1,2,4-triazole

To N-benzyloxymethyl-5-(2-cyano-4-pyridyl)-3-(2-chloro-4-pyridyl)-1,2,4-triazole (1.36 g) were added toluene (8.8 mL) and 2-propanol (8.8 mL), and the mixture was stirred. To the resulting solution was added p-toluenesulfonic acid monohydrate (3.50 g), and the resulting mixture was stirred for 18 hours at 80°C. The solvent was distilled away under reduced pressure, and the resulting residue was purified by column chromatography on silica gel (chloroform:methanol=20:1) to give 0.82 g of the captioned compound as white crystals

¹H NMR (DMSO-d₆) δ (ppm) : 8.03 (dd, 1H, J=5.13, 1.08Hz), 8.09 (d, 1H, J=1.08Hz), 8.31 (dd, 1H, J=4.86, 1.62Hz), 8.55 (d, 1H, J=1.62Hz), 8.63 (d, 1H, J=5.13Hz), 8.94 (d, 1H, J=4.86Hz)

### Example 18

### Preparation of 5-(2-cyano-4-pyridyl)-3-(2-phenyl-4-pyridyl)-1,2,4-triazole p-toluenesulfonate

To N-benzyloxymethyl-5-(2-cyano-4-pyridyl)-3-(2-phenyl-4-pyridyl)-1,2,4-triazole (1.00 g) were added toluene (6,5 mL) and 2-propanol (6.5 mL), and the mixture was stirred. To the resulting solution was added p-toluenesulfonic acid monohydrate (1.16 g) and the resulting mixture was stirred for 15 hours at 80°C. The mixture was brought to room temperature, and the precipitated white crystals were filtered, washed with 2-propanol and dried under reduced pressure to give 0.96 g of the captioned compound as white crystals.

¹H NMR (DMSO- d₆) δ (ppm) : 2.29 (s, 3H), 7.12 (d, 2H, J=8.10Hz), 7.49 (d, 2H, J=8.10Hz), 7.55-7.64 (m, 3H), 8.08 (dd, 1H, J=5.27, 1.62Hz), 8.15 (dd, 2H, J=8.10, 1.62Hz), 8.35 (dd, 1H, J=5.13, 1.89Hz), 8.58 (dd, 1H, J=1.62, 0.81Hz), 8.61 (dd, 1H, J=1.89, 0.81Hz), 8.91-8.95 (m, 2H)

Example 19

### Preparation of 5-(2-cyano-4-pyridyl)-3-[3-cyano-4-{2-(2-methoxyethoxy)ethoxy}phenyl]-1,2,4-triazole

To N-benzyloxymethyl-5-(2-cyano-4-pyridyl)-3-[3-cyano-4-{2-(2-methoxyethoxy)ethoxy}phenyl]-1,2,4-triazole (1.11 g) were added toluene (7.2 mL) and 2-propanol (7.2 mL), and the mixture was stirred. To the resulting solution was added p-toluenesulfonic acid monohydrate (3.36 g), and the resulting mixture was stirred for 30 hours at 80°C. The solvent was distilled away under reduced pressure, and the resulting residue was purified by column chromatography on silica gel (chloroform:methanol=20:1), further purified by column chromatography on silica gel (ethyl acetate), and dried under reduced pressure to give 0.39 g of the captioned compound as white crystals.
¹H NMR(DMSO-d₆) δ (ppm) : 3.25 (s, 3H), 3.46-3.49 (m, 2H), 3.63-3.66 (m, 2H), 3.83 (t, 2H, J=4.59Hz), 4.38 (t, 2H, J=4.59Hz), 7.51 (d, 1H, J=8.91Hz), 8.28-8.37 (m, 3H), 8.52 (s, 1H), 8.89 (d, 1H, J=5.13Hz)

### Test Example

### Serum uric acid level-lowering action

To male wister rats of 7 weeks old (4 rats per group) was orally administered compulsorily a test substance suspended in 0.5% aqueous solution of methyl cellulose (MC) in such an amount as to provide 0.3mg/5mL/Kg by using a stomach sonde. Six hours after the administration of the test substance, a blood sample was collected from the orbital vein. After allowing to stand at room temperature for 1 hour, the blood sample was centrifuged at 2000X g for 10 minutes to collect serum. A serum uric acid level was measured by using a kit for measuring uric acid (Wako Chemicals, phosphotungstic acid method) and a lowering rate of serum uric acid level was calculated according to the following equation to obtain 51.1% for the compound obtained in Example 2.

Lowering rate of serum uric acid level (%) =(1-average serum uric acid level of the group administered with test compound/average serum uric acid level of the group administered with MC)×100

## Claims

1. A 1,2,4-triazole compound represented by formula (a): 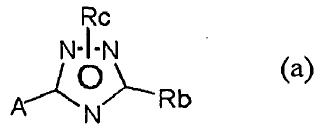 wherein A represents a pyridine N-oxide-4-yl group optionally substituted at a position other than 2-position or an optionally substituted 2-cyanopyridin-4-yl group, Rb represents an optionally substituted pyridyl or phenyl group, and Rc represents a group which makes the compound of formula (a) soluble in an organic solvent and which can be removed by an acid and which is bonded to any of the nitrogen atoms in the triazole ring, or a salt or hydrate thereof.

2. The 1,2,4-triazole compound, or the salt or hydrate thereof according to claim 1, wherein Rc is a group represented by formula (6)
-CH₂ORy (6)
(wherein Ry represents a substituted or unsubstituted alkyl group), or diphenylmethyl or a p-alkoxybenzyl group.

3. The 1,2,4-triazole compound, or the salt or hydrate thereof according to claim 1 or 2, wherein the salt is p-toluenesulfonate, methanesulfonate, hydrochloride or sulfate.

4. A process for producing a compound represented by formula (3): 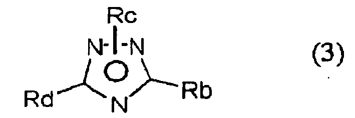 wherein Rd represents a pyridine N-oxide-4-yl group optionally substituted at a position other than 2-position, Rb represents an optionally substituted pyridyl or phenyl group, and Rc represents a group which makes the compound of formula (3) soluble in an organic solvent and which can be removed by an acid and which is bonded to any of the nitrogen atoms in the triazole ring, and a salt or hydrate thereof, which comprises reacting a compound represented by formula (1): 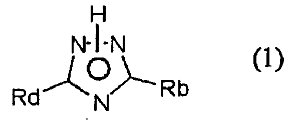 wherein Rd and Rb are as.defined above and the hydrogen atom is bonded to any of the nitrogen atoms in the triazole ring with a compound represented by formula (2):
Rc-X (2)
wherein Rc represents a group which makes the aimed compound soluble in an organic solvent and which can be removed by an acid and X represents a halogen atom or sulfonate residue.

5. A process for producing a compound represented by formula (4): 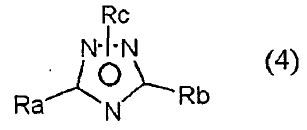 wherein Ra represents an optionally substituted 2-cyanopyridin-4-yl group, Rb represents an optionally substituted pyridyl or phenyl group, and Rc represents a group which makes the compound of formula (3) soluble in an organic solvent and which can be removed by an acid and which is bonded to any of the nitrogen atoms in the triazole ring, and a salt or hydrate thereof, which comprises reacting a compound represented by formula (3): 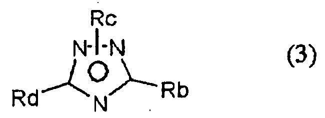 wherein Rd represents a pyridine N-oxide-4-yl group optionally substituted at a position other than 2-position, and Rb and Rc are as defined above with a nitrilization agent.

6. A process for producing a compound represented by formula (4): 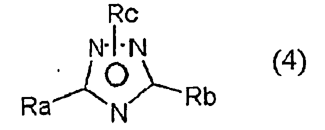 wherein Ra represents an optionally substituted 2-cyanopyridin-4-yl group, Rb represents an optionally substituted pyridyl or phenyl group, and Rc represents a group which makes the compound of formula (3) soluble in an organic solvent and which can be removed by an acid and which is bonded to any of the nitrogen atoms in the triazole ring, and a salt or hydrate thereof, which comprises reacting a compound represented by formula (1): 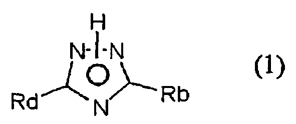 wherein Rd represents a pyridine N-oxide-4-yl group optionally substituted at a position other than 2-position, Rb is as defined above and the hydrogen atom is bonded to any of the nitrogen atoms in the triazole ring with a compound represented by formula (2):
Rc―X (2)
wherein Rc represents a group which makes the aimed compound soluble in an organic solvent and which can be removed by an acid, and X represents a halogen atom or sulfonate residue to give a compound represented by formula (3) : 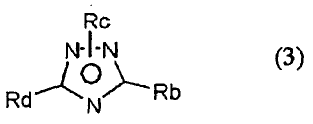 wherein Rb, Rc and Rd are as defined above, and then reacting the resulting compound (3) with a nitrilization agent.

7. A process for producing a compound represented by formula (5): 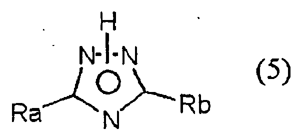 wherein Ra represents an optionally substituted 2-cyanopyridin-4-yl group, Rb represents an optionally substituted pyridyl or phenyl group, and the hydrogen atom is bonded to any of the nitrogen atoms in the triazole ring, and a salt or hydrate thereof, which comprises reacting a compound represented by formula (1): 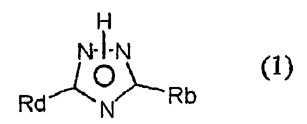 wherein Rd represents a pyridine N-oxide-4-yl group optionally substituted at a position other than 2-position, Rb is as defined above and the hydrogen atom is bonded to any of the nitrogen atoms in the triazole ring with a compound represented by formula (2):
Rc-X (2)
wherein Rc represents a group which makes the aimed product soluble in an organic solvent and which can be removed by an acid, and X represents a halogen atom or sulfonate residue to give a compound represented by formula (3): 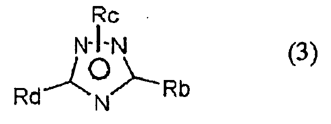 wherein Rc represents a group which makes the compound of formula (3) soluble in an organic solvent and which can be removed by an acid and which is bonded to any of the nitrogen atoms in the triazole ring, and Rb and Rd are as defined above, and then reacting the resulting compound (3) with a nitrilization agent to give a compound represented by formula (4): 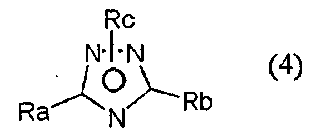 wherein Ra, Rb and Rc are as defined above, and further reacting the resulting compound with an acid.

8. A process for producing a compound represented by formula (5): 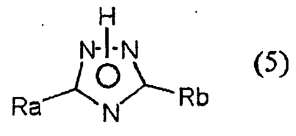 wherein Ra represents an optionally substituted 2-cyanopyridin-4-yl group, Rb represents an optionally substituted pyridyl or phenyl group, and the hydrogen atom is bonded to any of the nitrogen atoms in the triazole ring, and a salt or hydrate thereof, which comprises reacting a compound represented by formula (3): 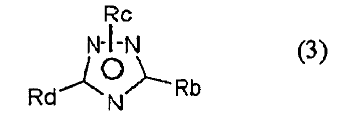 wherein Rd represents a pyridine N-oxide-4-yl group optionally substituted at a position other than 2-position, Rc represents a group which makes the compound of formula (3) soluble in an organic solvent and which can be removed by an acid and which is bonded to any of the nitrogen atoms in the triazole ring, and Rb is as defined above with a nitrilization agent to give a compound represented by formula (4): 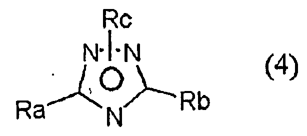 wherein Ra, Rb and Rc are as defined above, and then reacting the resulting compound with an acid.

9. The process according to any one of claims 4-8, wherein Rc is a group represented by formula (6):
-CH₂ORy (6)
(wherein Ry represents a substituted or unsubstituted alkyl group), or diphenylmethyl or a p-alkoxybenzyl group.

10. The process according to any one of claims 4-9,
wherein the salt is p-toluenesulfonate, methanesulfonate, hydrochloride or sulfate.
